# EUROPEAN PATENT SPECIFICATION

(11) **EP 1 395 333 B1**
(45) Date of publication and mention of the grant of the patent: **31.12.2008**
(21) Application number: 02743627.8
(22) Date of filing: 04.06.2002
(51) Int. Cl.: A61N 1/32

(54) **ELECTROPORATION DEVICE WHICH REDUCES MUSCLE CONTRACTION AND PAIN SENSATION**
ELEKTROPORATIONSEINRICHTUNG ZUR REDUZIERUNG DER KONTRAKTION VON MUSKELN UND DER SCHMERZEMPFINDUNG
DISPOSITIF D'ELECTROPORATION REDUISANT LA CONTRACTION MUSCULAIRE ET LA SENSIBILITE A LA DOULEUR

(30) Priority: 04.06.2001 IT TO20010534
(43) Date of publication of application: 10.03.2004
(73) Proprietor: IGEA S.p.A., 41012 Carpi (IT)
(72) Inventor: MIKLAVCIC, Damijan c/o University of Ljubljana, 1000 Ljubljana (SI); MIR, Lluis c/o Institut Gustave-Roussy, UMR 8532 CNRS, F-94805 Villejuif Cedex (FR)
(74) Representative: Bongiovanni, Simone
(86) International application number: PCT/IT2002/000361
(87) International publication number: WO 2002/098504

(56) References cited:
- WO-A-01/80946
- WO-A-99/52589
- US-A- 4 226 246
- US-A- 6 068 650

## Description

### TECHNICAL FIELD

The present invention relates to an electroporation device and method.

### BACKGROUND ART

As is known, recent biological, microbiological and pharmacological applications involve introducing molecules into cells, which is done by introducing the molecules through the cell membranes.

Alternatively the introduction may be done by exposing cells to electric pulse, thus enabling transport of molecules through the cell membrane.

The molecules may be inorganic substances (e.g. drugs) or organic molecules (DNA molecules for example are known to be introduced in cells).

Molecules are introduced using various methods, including:
viral vectoring: associating the molecule with a virus, which is then introduced into the cell;
chemical vectoring : associating the molecule with a chemical substance for reducing the resistance of the cell membrane and so permitting introduction of the molecule into the cell; and
ballistic methods : accelerating the molecule so that it strikes and penetrates the cell membrane.

Known methods involve several drawbacks, including: risk of immunity reaction to the vector; production difficulties and poor stability of the vector itself (viral vectoring); ineffectiveness, toxicity and poor selectivity (chemical vectoring). As for ballistic methods, these only apply to surface cells.

New so-called electroporation methods have recently been devised, which are based on the application of electric pulses to the cells in order to produce an electric field that permeabilizes the cell structure enabling the substances to cross the cell membrane.

The above methods normally provide short pulses delivered at relatively low repetition frequency (for instance in the field of electro-chemo-therapy it is known to apply one or more pulses (for instance 1, 2, 4, 6 or 8 pulses) having time width of 100 µs and 1Hz repetition frequency) or provide longer pulses (for instance it is known to apply pulses having time width of some miliseconds for treating cells with DNA).

In the above cases, the underlying nerves and muscles of the patient (man or animal) who receives the pulses are excited, resulting in nerve excitation / muscle contraction and pain perception. The result is an unpleasant sensation for the patient that strongly limits the application of the above pulses for a longer period of time in case of treating large volumes of tissue with e.g. multiple needle electrodes arranged in an array.

However, not only present protocols use several electric pulses (resulting in the equivalent number of disagreeable sensations by the patient and in the equivalent number of muscle contractions), but studies both in vitro and in vivo have shown that better electropermeabilization of the cells is achieved when several pulses are delivered. Indeed, for the same total duration of the electric field delivery, several short pulses (e.g. 10 pulses of 100 microseconds; total duration = 1 millisecond) lead to a better permeabilization and drug uptake than a single pulse of 1 millisecond. Documents US-A-6068 650 and WO 99/52 589 disclose such electroporations devices. The features of the first part of the revendication 1 are known from US-A-6068650.

### DISCLOSURE OF THE INVENTION

It is an object of the present invention to provide an electroporation device and method designed to eliminate the drawbacks of known electroporation devices and methods.

In particular, it is an object of the present invention to provide an electroporation device that produces pulses that will strongly limit the sensations felt by the patients to a single one, that is to the sensation and contraction produced by the first pulse of the train of pulses. Under (these) proposed conditions, treatment can be finished before sensation has been felt by the patient. Moreover, the reduction in the number of contractions provoked by the treatment could potentially decrease muscle structure alteration, or other injuries to the muscles besides those strictly linked to the cell permeabilization.

According to the present invention, there is provided an electroporation device as described in Claim 1.

The present invention also relates to an electroporation method as described in Claim 7.

### BRIEF DESCRIPTION OF THE DRAWINGS

A preferred, non-limiting embodiment of the invention will be described by way of example with reference to the accompanying drawings, in which:
Figure 1 shows, schematically, an electroporation device in accordance with the teachings of the present invention; and
Figures 2a, 2b, 2c, 2d and 2e show a signal produced by the Figure 1 device.

### BEST MODE FOR CARRYING OUT THE INVENTION

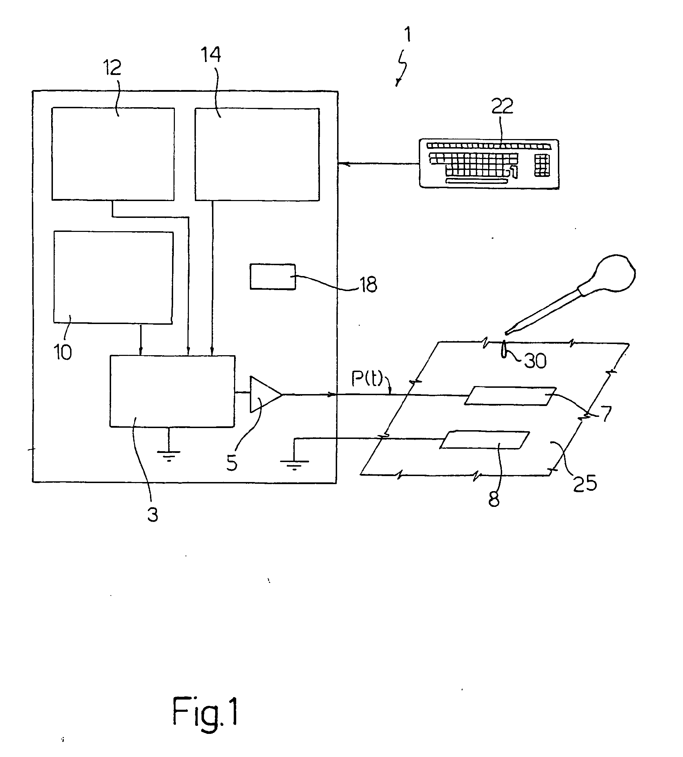

Number 1 in Figure 1 indicates as a whole an electroporation device.

Device 1 comprises a signal generator, in particular a pulse generator 3 for producing a train of pulses, having an output connected to an input of a power amplifier 5 having its output communicating with a couple of electrodes 7 and 8.

The pulse generator 3 is able to produce a train of pulses P(t) whose shape, amplitude and repetition is dependant on a plurality of information signals received at inputs of the pulse generator 3.

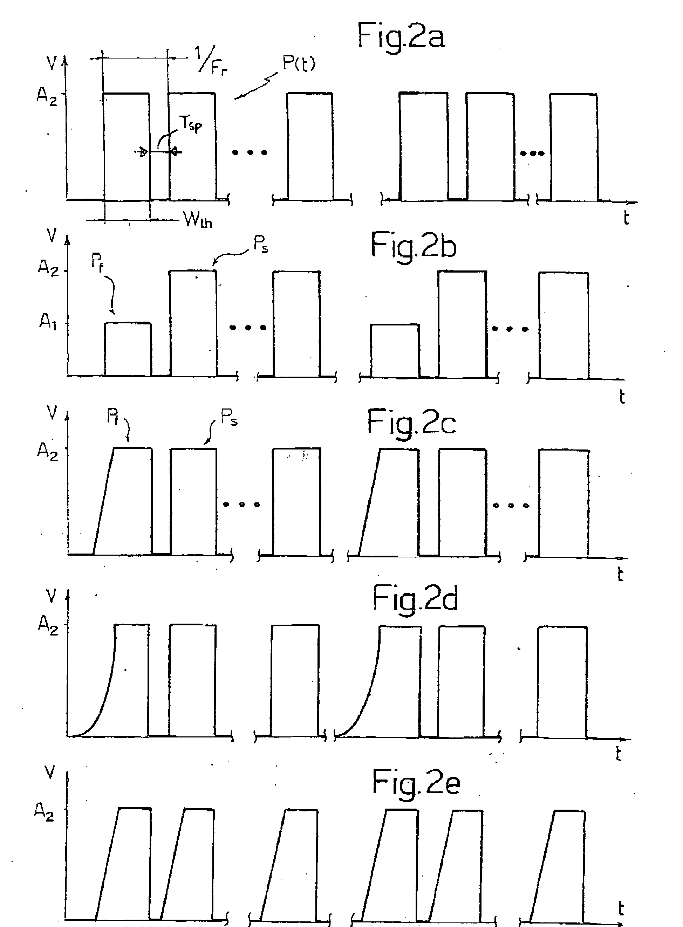

In particular, the pulse generator 3 co-operates with:
◆ a first circuit 10 that is designed to control the time width Wth (figure 2a) of each generated pulse P(t) - in particular, the time width may be regulated continuously between about 10 microseconds and 10 milliseconds and, more practically, between about 30µs and 250µs in preferred embodiments of the trains of pulses;
◆ a second circuit 12 that is designed to control the frequency of repetition Fr of the generated pulses P(t) of the train - in particular, the frequency of repetition Fr being variable continuously (or in steps, increments) between about 1 KHz and 100 KHz and more practically between about 2 kHz and 25 kHz in preferred embodiment of the train of pulses. The frequency of repetition Fr may increases with the decrease of the time width Wth and decreases with the increase of the time width Wth even if the frequency of repetition and the time width do not need to be inversely proportional (the delay between two consecutive pulses and pulse duration can be fixed independently).
   For instance for 30µs pulses the frequency of repetition is conveniently 17 kHz and for 100µs pulses the frequency of repetition is conveniently 5 or 3,2 kHz; and
◆ a third circuit 14 that regulates the amplitude of each pulse applied to the electrodes 7,8 - such amplitude being adjustable between 50 Volt and 2000 Volt., preferably below 1100 Volts, to the purposes of the treatment (Electrochemotherapy or electrogenetherapy, i.e. DNA electrotransfer for gene therapy), to the tissues, and to the distance between the electrodes.

Conveniently the pulses are rectangular pulses, even if it is clear that pulses having a different shape may be used (for instance triangular pulses, trapezoidal, monopolar or bipolar pulses, sinusoidal pulses,...).

The pulse generator 3 and circuits 10, 12 and 14 are controlled by a central processing unit (CPU) 18 receiving commands from the exterior (for instance commands introduced by means of a keyboard 22) so that a train of pulses P(t) having particular width, frequency of repetition and amplitude may be generated.

In actual use, electrodes 7,8 are applied to a tissue portion 25 (shown schematically in Figure 1) containing live cells. The tissue portion may be preferably one forming part of a live being (human or animal).

Tissue portion 25 is also applied with a substance (organic or inorganic or biopolymeric) 30 to be introduced into the cells. The substance 30 may be applied in a number of different ways, some of which are listed below by way of non-limiting examples:
- direct application of the substance to the tissue portion, e.g. by applying the tissue portion with a fluid containing the substance;
- indirect application of the substance, e.g. by introducing the substance into the circulatory system of the tissue portion; and
- injecting the substance, e.g. using needle-like electrodes 7,8, each having an inner conduit containing the substance to be injected into the tissue portion. The substance may also be injected using needles separate from the electrodes.

The substance 30 introduced may be inorganic or organic or biopolymeric, e.g.
- a nucleic acid;
- a DNA molecule containing regulatory sequences and sequence coding for therapeutic genes or genes of interest for biomedical or biotechnological purposes;
- an oligonucleotide, whether natural (phosphodiesters) or modified (inside the backbone of the oligonucleotide, such as phosphosulfates, or at the extremities, by addition of groups to protect the oligonucleotides from digestion of nucleoasis; the description of oligonucleotide modifications being non-limiting);
- a protein or peptide, whether natural or genetically or chemically modified, extracted from natural sources or obtained by synthesis, or a molecule simulating the structure of a protein or peptide, whatever its structure;
- a cytotoxic agent, in particular, the antibiotic bleomycin or cisplatinum;
- a penicillin; and
- a pharmacological agent other than a nucleic acid.

Device 1 is activated to generate a train of pulses that are spaced one with respect the other of a time interval Tsp that is lower than the refractory period of the nerves and/or muscles present in tissue portion 35.

More precisely the refractory period is divided in two parts: the absolute refractory period (during which the membrane of the nerve/muscle can not be depolarised i.e. second action potential can not be generated) and relative refractory period (during which new action potential can be generated, the membrane is depolarised, but only with stronger electric pulse).

Numerical values of refractory period for tissue portion 25 slightly vary depending of the type of nerve and muscle contained in tissue portion 25.

In general, myelinated nerve fibres have shorter refractory periods than unmyelinated, and nerve fibres with larger diameters have shorter refractory periods than thinner nerve fibres.

Some examples of the refractory period are the following:
◆ Example 1: absolute refractory period (ARD) = 0.4 ms, relative refractory period (RRP)= 0.1 to 0.2 ms, thus the refractory period (being the sum of both) equals 0.5 to 0.6 ms. These values are given for large myelinated nerve fibres in humans and are thus the shortest.
◆ Example 2: ARD = 0.5 to 1 ms, RRD = 0.5 to 2 ms, thus the total refractory period being the sum of both equals 1 to 2.5 ms.
◆ Example 3: ARD = 1 ms, RRD = 10 to 15 ms.

The following table also provides examples of refractory periods for nerves having different diameters (minimum/Maximum) and different composition (Unmmyelinated and Myelinated).

| | Unmmyelinated | Unmmyelinated | Myelinated | Myelinated |
|---|---|---|---|---|
| Diameter of nerve fibre | Minimum | maximum | minimum | Maximum |
| Refractory period | 2 ms | 2 ms | 1.2 ms | 0.4 ms |

According to the present invention, the user feels only the first pulse of the train and does not feel anymore (or to a considerably lesser extent) the successive pulses as the nerve and/or the muscle, once activated for a first time, has not time to react to the following pulses and is automatically disposed in a state in which the following pulses are not sensed.

In other words, the device of the present invention generates a train of pulses having a frequency Fr (1 KHz - 100 KHz) that is in any case higher than the maximum frequency of action potential of the nerve and/or the muscle tissue present in tissue portion 25. In fact, the frequency of action potential of the nerves and/or skeletal muscles extends from 400 Hz to 2,5 KHz and therefore the frequency of repetition of the generated pulses is higher than the frequency of action potential of the nerves and/or muscles. As a consequence, the nerves and/or muscles are not activated with each consecutive pulse and the patient does not suffer additional and/or excessive pain and/or has not unpleasant sensations, but the first one.

As above stated, the patient only feels the application of the first pulse and does not feel anymore the consecutive pulses of the train; in order to minimise the disturbance inflicted to a patient, according to one embodiment of the invention, the first pulse Pf (figure 2b) of the train has an amplitude A1 that is lower than the amplitude A2 of the following pulses Ps of the train so that the first pulse that activates the muscles (and that is potentially sensed) does not substantially induce pain due to its negligible amplitude and the following pulses (that are not sensed due their time spacing) have a larger amplitude in order to achieve a good permeabilization in the substrate.

According to an alternative embodiment shown in figure 2c, the first pulse Pf has a leading front in the form of a linear ramp ranging from 0 Volt to amplitude A2 and the successive pulses Ps are rectangular and have fixed amplitude A2.

According to the embodiment shown in figure 2d, the leading front of the fist pulse Pf is gradually increasing by other than linear function, for instance following an exponential function. Further pulses Ps are rectangular pulses having fixed amplitude A2.

According to the embodiment shown in figure 2e all the pulses Pf, Ps of the train have a leading front increasing gradually (linearly or exponentially) from 0 to amplitude A2.

The knowledge gathered by the Applicant indicates that applying pulses with the above range of time width and the above frequency of repetition permits an excellent electroporation of the cells and at the same time does not induce action potential in the nerves and/or muscles with each consecutive electroporation pulse so that the patient does not suffer additional and/or excessive pain and/or has not unpleasant sensations but the first one.

Clearly, changes may be made to the device as described herein without, however, departing from the scope of the present invention.

## Claims

1. An electroporation device comprising pulses generating means (3) connectable (5) to electrodes (7,8) fittable to a substrate (25) of a live being comprising cells; said electrodes (7,8) producing, in said substrate (35), an electric field which induces permeabilization of the membranes of said cells to facilitate introduction of substances (30) into the cells,
**characterized by** comprising frequency regulating means (12) designed to produce a train of pulses that are spaced one with respect the other of a time interval Tsp that is lower than the refractory period of the nerves and/or muscles present in the substrate (25).

2. An electroporation device as claimed in claim 1, wherein the frequency regulating means (12) control the frequency of repetition Fr of the generated pulses in order to vary such a frequency of repetition between about 1 KHz and 100 KHz.

3. An electroporation device as claimed in claim 1 or 2, wherein the frequency regulating means (12) control the frequency of repetition Fr of the generated pulses in order to vary such a frequency of repetition between about 2 KHz and 25 KHz.

4. Electroporation device as claimed in any of the foregoing claims, wherein the device comprises time amplitude regulating means (10) designed to control the time width Wth of each generated pulse P(t); said time width Wth being regulated between about 10µs and 10 ms.

5. Electroporation device as claimed in any of the proceedings claims, wherein the device comprises time amplitude regulating means (10) designed to control the time width Wth of each generated pulse P(t); said time width Wth being regulated between about 30µs and 250µs.

6. Electroporation device as claimed in any one of the foregoing claims, wherein the first pulse Pf of the train has an amplitude A1 that is lower than the amplitude A2 of the following pulses Ps.

7. Electroporation device as claimed in any one of the foregoing claims, wherein the first pulse Pf of the train has a leading front monotonically increasing from 0 Volt to an amplitude A2.

## Patentansprüche

1. Eine Elektroporationsvorrichtung, umfassend Puls erzeugende Mittel (3), welche mit Elektroden (7, 8) verbindbar (5) sind, welche an ein Substrat (25) eines Lebewesens, welches Zellen umfasst, anpassbar sind; wobei die Elektroden (7, 8) in dem Substrat (35) ein elektrisches Feld erzeugen, welches eine Permeablisierung der Membranen der Zellen veranlasst, um ein Einführen von Substanzen (30) in Zellen zu vereinfachen,
**dadurch gekennzeichnet, dass** sie Frequenz regulierende Mittel (12) umfasst, welche gestaltet sind, um eine Abfolge von Pulsen zu erzeugen, welche voneinander ein Zeitintervall Tsp entfernt sind, welches geringer ist als die refraktäre Periode der Nerven und/oder Muskeln, welche in dem Substrat (25) vorhanden sind.

2. Elektroporationsvorrichtung nach Anspruch 1, worin die Frequenz regulierenden Mittel (12) die Frequenz oder Wiederholung Fr der erzeugten Pulse steuern, um eine solche Wiederholungsfrequenz zwischen 1 KHz und 100 KHz zu variierten.

3. Elektroporationsvorrichtung nach Anspruch 1 oder 2, worin die Frequenz regulierenden Mittel (12) die Wiederholungsfrequenz Fr der erzeugten Pulse steuern, um eine solche Wiederholungsfrequenz zwischen etwa 2 KHz und 25 KHz zu variieren.

4. Elektroporationsvorrichtung nach einem der vorhergehenden Ansprüche, worin die Vorrichtung Zeitamplituden regulierende Mittel (10) umfasst, welche entworfen sind, um die Zeitbreite Wth von jedem erzeugten Puls P(t) zu steuern; wobei die Zeitbreite Wth zwischen etwa 10 µs und 10 ms reguliert wird.

5. Elektroporationsvorrichtung nach irgendeinem der vorhergehenden Ansprüche, worin die Vorrichtung Zeitamplituden regulierende Mittel (10) umfasst, welche entworfen sind, um die Zeitbreite Wth von jedem erzeugten Puls P(t) zu steuern; wobei die Zeitbreite Wth zwischen 30 µs und 250 µs reguliert wird.

6. Elektroporationsvorrichtung nach irgendeinem der vorhergehenden Ansprüche, worin der erste Puls Pf der Abfolge eine Amplitude A1 aufweist, welche geringer ist als die Amplitude A2 des folgenden Pulses.

7. Elektroporationsvorrichtung nach einem der vorhergehenden Ansprüche, worin der erste Puls Pf der Abfolge von Pulsen eine führende Stirnseite aufweist, welche monoton von 0 Volt zu einer Amplitude A2 ansteigt.

## Revendications

1. Dispositif d'électroporation comprenant des moyens (3) générant des impulsions pouvant être raccordés (5) à des électrodes (7, 8) pouvant être fixées sur un substrat (25) d'un être vivant comprenant des cellules ; lesdites électrodes (7, 8) produisant, dans ledit substrat (35), un champ électrique qui induit une perméabilisation des membranes desdites cellules pour faciliter l'introduction des substances (30) dans les cellules,
**caractérisé par** le fait de comprendre des moyens de régulation de fréquence (12) conçus pour produire une succession d'impulsions qui sont espacées les unes des autres à un intervalle de temps Tsp qui est inférieur au temps de réfraction des nerfs et/ou muscles présents dans le substrat (25).

2. Dispositif d'électroporation selon la revendication 1, dans lequel les moyens de régulation de fréquence (12) contrôlent la fréquence de répétition Fr des impulsions générées afin de faire varier une telle fréquence de répétitions entre environ 1 KHz et 100 KHz.

3. Dispositif d'électroporation selon la revendication 1 ou 2, dans lequel les moyens de régulation de fréquence (12) contrôlent la fréquence de répétition Fr des impulsions générées afin de faire varier une telle fréquence de répétitions entre environ 2 KHz et 25 KHz.

4. Dispositif d'électroporation selon l'une quelconque des revendications précédentes, dans lequel le dispositif comprend des moyens de régulation de l'amplitude temporelle (10) conçus pour contrôler le délai de temporisation Wth de chaque impulsion générée P(t) ; ledit délai de temporisation Wth étant régulé entre environ 10 µs et 10 ms.

5. Dispositif d'électroporation selon l'une quelconque des revendications précédentes, dans lequel le dispositif comprend des moyens de régulation d'amplitude temporelle (10) conçus pour contrôler le délai de temporisation Wth de chaque impulsion générée P(t) ; ledit délai de temporisation Wth étant régulé entre environ 30 µs et 250 µs.

6. Dispositif d'électroporation selon l'une quelconque des revendications précédentes, dans lequel la première impulsion Pf de la succession d'impulsions possède une amplitude A1 qui est inférieure à l'amplitude A2 des pulsations suivantes Ps.

7. Dispositif d'électroporation selon l'une quelconque des revendications précédentes, dans lequel la première impulsion Pf de la succession d'impulsions possède un front d'attaque augmentant de façon monotonique de 0 volt à une amplitude A2.
